# EUROPEAN PATENT SPECIFICATION

(11) **EP 0 705 822 B1**
(45) Date of publication and mention of the grant of the patent: **12.07.2000**
(21) Application number: 95115552.2
(22) Date of filing: 02.10.1995
(51) Int. Cl.: C07D 233/78, C07D 233/80, G03C 7/36

(54) **Hydantoin-substituted acylacetanilide compounds and method of manufacturing the same**
Hydantoin-substituierte Acylacetanilid Verbindungen und Verfahren zu deren Herstellung
Dérivés d'acyleacétamine hydantoin substitués et procédé pour leur préparation

(30) Priority: 03.10.1994 JP 23922794
(43) Date of publication of application: 10.04.1996
(73) Proprietor: Fuji Photo Film Co., Ltd., Kanagawa-ken (JP)
(72) Inventor: Yamakawa, Katsuyoshi, Minami-ashigara-shi, Kanagawa-ken (JP); Sato, Tadahisa, Minami-ashigara-shi, Kanagawa-ken (JP); Hanaki, Koichi, Minami-ashigara-shi, Kanagawa-ken (JP)
(74) Representative: Hansen, Bernd, Dr. Dipl.-Chem.

(56) References cited:
- EP-A- 0 041 170
- EP-A- 0 679 634
- EP-A- 0 711 758
- GB-A- 1 113 038
- GB-A- 2 066 811
- US-A- 2 801 171
- RESEARCH DISCLOSURE, vol. 164, December 1977 pages 47-49, MONBALIU M. & VAN POUCKE R. '16451. Preparation of amino-substituted phenones'

## Description

The present invention relates to a novel hydantoin-substituted acylacetanilide compound that is useful as an intermediate of the yellow coupler for use in a silver halide color photographic light-sensitive material, and to a method of manufacturing the compound.

As a yellow coupler for use in color photography, generally used compounds are composed of a pivaroylacetanilide skeleton or a benzoylacetanilide skeleton, as a basic skeleton, and a 5-membered heterocyclic ring, such as hydantoin, located at a reaction point of the compound in a dye-forming reaction (see, for example, JP-B ("JP-B" means examined Japanese patent publication) Nos. 10739/1983, 45135/1981, and 44420/1981). In order to improve the efficiency of the dye-forming reaction and to optimize the hue of the formed dye, further investigation was conducted for optimizing a substituent at the part of anilide, which resulted in a 2-chloro(or alkoxy)-5-acylamino-type coupler represented by general formula (A). 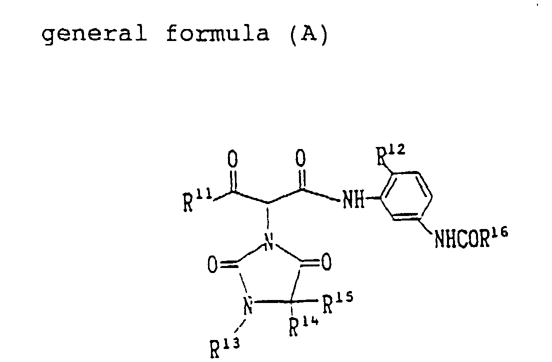 wherein R¹¹ represents a tert-butyl group or a p-alkoxyphenyl group, R¹² represents a chlorine atom or an alkoxy group, R¹³ represents a hydrogen atom, an alkyl group, a benzyl group, an alkylsulfonyl group, or an arylsulfonyl group, R¹⁴ represents a hydrogen atom or an alkyl group, and R¹⁵ represents an alkyl group or an alkoxy group, and R¹⁶ represents a substituted or unsubstituted alkyl group.

This compound is generally synthesized by the method illustrated below. That is, a β-ketoester compound 1, as a key intermediate, is reacted with aniline, to obtain an acylacetanilide compound 2. The compound 2 is further subjected to halogenization, to obtain compound 3, followed by a substitution reaction, to obtain a yellow coupler of general formula (A).

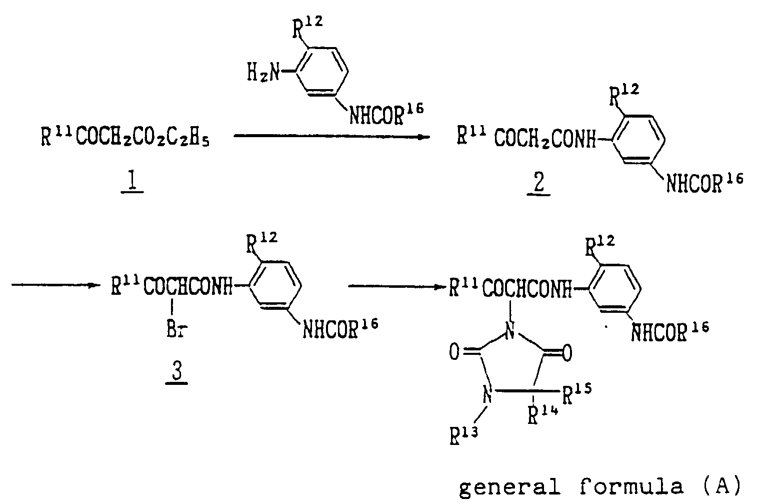

However, this method has several problems, as described below. For example, during the course of the substitution reaction of the compound 3 to obtain the yellow coupler of general formula (A), the compound 2 is sometimes obtained by reduction, because the compound 3 is an active halide substituted by two electron-attractive groups. Furthermore, the compound 3 provides a pKa value as low as that of hydantoin compound, and the compound 3 lowers reactivity of the hydantoin compound under a reaction conditions. Therefore, in order to complete the reaction, it is necessary to use a hydantoin compound represented by formula: 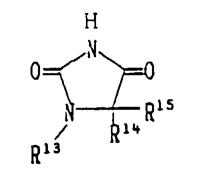 in an excess amount of the compound 3. Accordingly, it has been desired to resolve such problems.

GB-A-2 066 811 reveals acylacetanilide yellow couplers having at its acyl portion or anilide portion a radical of the formula: -X-Y-Z, wherein X is -NHCO- or -CONH-, Y is a divalent organic radical, Z is 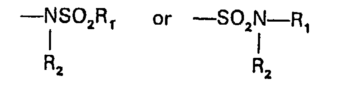 wherein R₁ is a substituted or non-substituted alkyl or aryl group, R₂ is a hydrogen atom or a substituted or unsubstituted alkyl or aryl group.

In US-A-280 1 171, GB-B-1 113 038, EP-A-0 041 170 and Research Disclosure (1977) 164, 47-49 some specific nitro-substituted acylacetanilides are described which, however, do not comprise any hydantoin group.

An object of the present invention is to provide a novel intermediate for manufacturing a yellow coupler.

Another object of the present invention is to provide a method for manufacturing the intermediate, which overcomes such problems in the production of color photographic yellow couplers, and which enables obtaining color photographic yellow couplers at a high yield in short steps.

A further object of the present invention is to provide a method for manufacturing color photographic yellow couplers in high purity.

The present inventors intensively investigated in order to overcome the problems in the above-described conventional method of producing yellow couplers. As a result, the present inventors have found that the compound represented by general formula (I) can be obtained, in a high yield, by a reaction of a substituted acetyl compound represented by general formula (III) (which is easily obtained by reacting a corresponding haloacetyl compound with an almost equimolar amount of a hydantoin compound) and a phenylurethane compound represented by general formula (IV), and then the compound of general formula (I) is subjected to reduction and acylation, whereby a yellow coupler represented by general formula (II) can be obtained in a high yield. 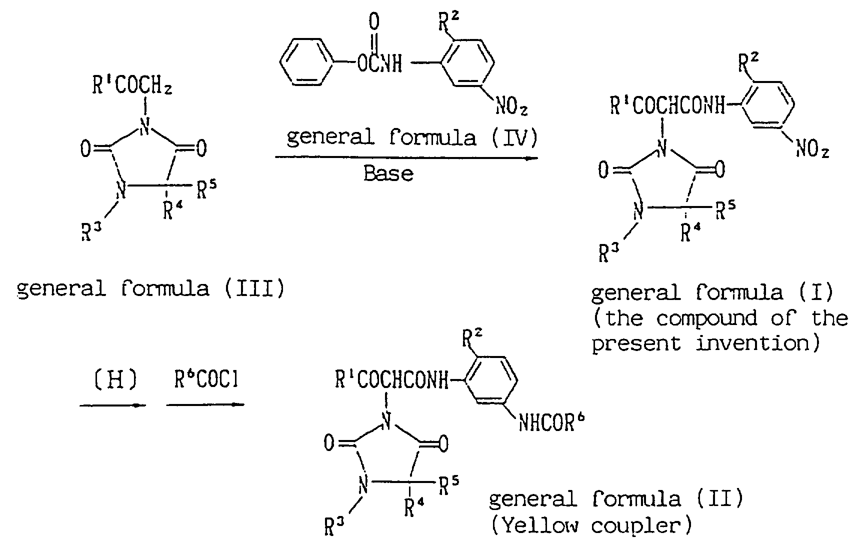 wherein R¹ represents a tert-butyl group or a p-alkoxyphenyl group, R² represents a chlorine atom or an alkoxy group, R³ represents a hydrogen atom, an unsubstituted or alkoxy-substituted alkyl group, an alkylsulfonyl group, or an arylsulfonyl group, R⁴ represents an alkyl group, R⁵ represents an alkyl group, and R⁶ represents a substituted or unsubstituted alkyl group.

That is, the present invention includes:
(1) a hydantoin-substituted acylacetanilide compound represented by general formula (I) 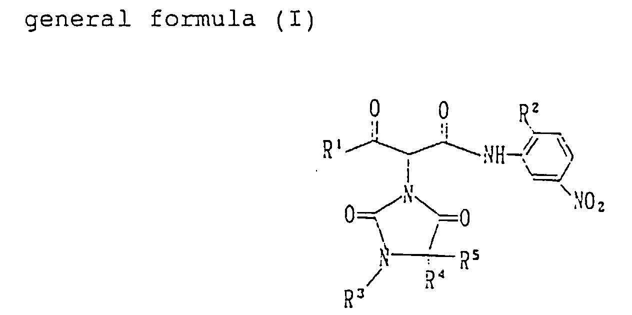 wherein R¹ represents a tert-butyl group or a p-alkoxyphenyl group, R² represents a chlorine atom or an alkoxy group, R³ represents a hydrogen atom, an unsubstituted or alkoxy-substituted alkyl group, an alkylsulfonyl group, or an arylsulfonyl group, R⁴ represents an alkyl group, and R⁵ represents an alkyl group, and
(2) a method of manufacturing a hydantoin-substituted acylacetanilide compound represented by general formula (I), comprising reacting a hydantoin-substituted acetyl compound represented by general formula (III) with a urethane compound represented by general formula (IV) in the presence of a base 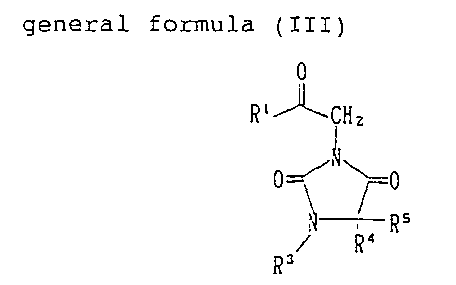 wherein R¹, R³, R⁴, and R⁵ each have the same meanings as those of general formula (I) 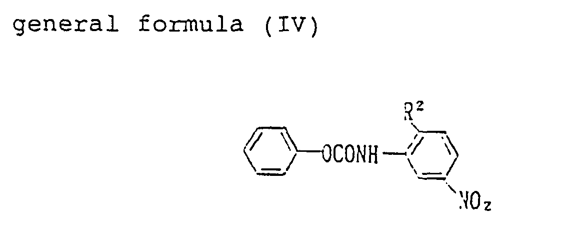 wherein R² has the same meaning as that of general formula (I)

R¹ represents a tert-butyl group, or a phenyl group having at the para position an alkoxy group that preferably contains 1 to 18 carbon atoms, more preferably 1 to 4 carbon atoms (e.g., methoxy, ethoxy, octoxy, hexadecoxy, octadecoxy). R¹ preferably represents a tert-butyl group or a p-methoxyphenyl group, and particularly preferably it represents a tert-butyl group.

R² represents a chlorine atom, or an alkoxy group that preferably contains 1 to 18 carbon atoms, more preferably 1 to 4 carbon atoms (e.g., methoxy, ethoxy, octoxy, hexadecoxy, octadecoxy), and preferably R² represents a chlorine atom or a methoxy group.

R³ represents a hydrogen atom; an unsubstituted or alkoxy-substituted alkyl group that preferably contains 1 to 18 carbon atoms, more preferably 1 to 6 carbon atoms (e.g., methyl, ethyl, hexyl, octyl, octadecyl, methoxymethyl, methoxyethoxymethyl); an alkylsulfonyl group that preferably contains 1 to 18 carbon atoms, more preferably 1 to 4 carbon atoms (e.g., methanesulfonyl, butanesulfonyl, hexadecanesulfonyl); or an arylsulfonyl group that preferably contains 6 to 18 carbon atoms, more preferably 6 to 10 carbon atoms (e.g., benzenesulfonyl, p-toluenesulfonyl). Particularly preferably R³ represents a hydrogen atom, a methoxymethyl group, a methanesulfonyl group, a benzenesulfonyl group, or a p-toluensulfonyl group.

R⁴ represents an alkyl group that preferably contains 1 to 18 carbon atoms, more preferably 1 to 4 carbon atoms (e.g., methyl, ethyl, propyl, butyl, octyl, hexadecyl), and preferably R⁴ represents a methyl group.

R⁵ represents an alkyl group that preferably contains 1 to 18 carbon atoms, more preferably 1 to 4 carbon atoms (e.g., methyl, ethyl, propyl, butyl, octyl, hexadecyl), and preferably R⁵ represents a methyl group.

In the present invention, a combination in which R¹ represents a t-butyl group and R² represents a chlorine atom or a methoxy group, is preferred.

It is preferable that, when R⁴ represents an alkyl group, R⁵ represents an alkyl group, and particularly when R⁴ represents a methyl group, R⁵ represents a methyl group. Furthermore, it is preferred that, when R⁴ represents a methyl group, R³ represents a hydrogen atom, a methoxymethyl group, a methanesulfonyl group, a benzenesulfonyl group, or a p-toluenesulfonyl group.

The compound of the present invention can be obtained in a high yield by reacting a substituted acetyl compound represented by general formula (III) and a phenylurethane compound represented by general formula (IV) in the presence of a base.

The amount of the substituted-acetyl compound of the general formula (III) to be added is preferably 3 to 0.5 mol, and more preferably 2 to 0.8 mol per mol of the phenylurethane compound of the general formula (IV).

As the base, LDA (lithium diisopropylamide) and sodium hydride are used in an amount of 0.9 to 5 mol, and preferably 1 to 3 mol per mol of the compound represented by general formula (IV). Sodium hydride is particularly preferred.

As a reaction solvent, use can be made of ether-series solvents, such as ether and tetrahydrofuran; aromatic hydrocarbon-series solvents, such as toluene and xylene; and amide-series solvents, such as DMF, DMAc, DMI, and NMP.

A reaction temperature is generally from -20°C to 140°C, and preferably from 0 to 80°C.

The substituted acetyl compound represented by general formula (III) can be obtained in a high yield by reacting a corresponding α-haloacetyl compound with a compound of formula: 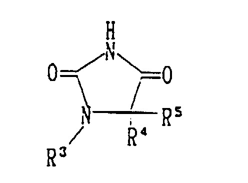 in an amount of 0.5 to 2 mol, and preferably from 0.9 to 1.1 mol per mol, of the α-haloacetyl compound in the presence of a base. The above method is characterized by an almost equimolar amount of hydantoins being used with the α-haloacetyl compound, whereby the substituted acetyl compound is obtained in a high yield.

As the base, use can be made of inorganic bases, such as sodium hydride, potassium tert-butoxide, sodium carbonate, potassium carbonate, sodium methoxide, sodium hydroxide, and potassium hydroxide; and organic bases, such as 1,8-diazabicyclo[5.4.0]undecene (DBU), N,N-diisopropylethylamine, and triethylamine. As a reaction solvent, use can be made of ether, tetrahydrofuran (THF), dioxane, acetonitrile, N,N-dimethylfolmamide (DMF), N,N-dimethylacetamide (DMAc), 1,3-dimethyl-2-imidazolidone (DMI), and 1-methyl-2-pyrrolidone (NMP). A reaction temperature is generally from 0 to 140°C, and preferably from 10 to 100°C.

The phenylurethane compound represented by general formula (IV) can be easily synthesized by reacting a corresponding aniline derivative and phenyl chlorocarbonate. As a solvent, use can be made of the same compounds as described in the preparation of the compound represented by general formula (III).

The compound of the present invention is useful as an intermediate of photographic yellow couplers as described below. 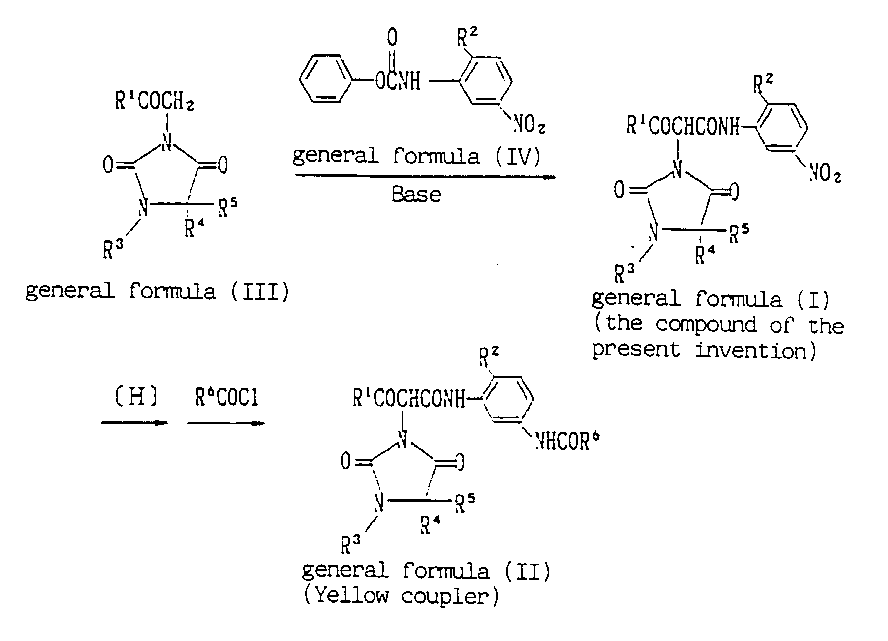 wherein R¹, R², R³, R⁴, R⁵, and R⁶ each have the same meanings as described above.

A nitro group of the compound of the present invention is reduced to an amino group. The resulting amino compound is reacted with various kinds of acid chlorides (R⁶COCl), to obtain, in a high yield, a yellow coupler represented by general formula (II).

In order to convert a nitro group to an amino group, various conditions hitherto known and described in, for example, Shinjikkenkagakukoza 14 [III] p. 1333, Maruzen (1975), can be applied. Examples of a typical reducing agent (reduction condition) are various metals, such as zinc, iron, tin, and stannous chloride; sulfides, such as sodium sulfide, sodium hydrosulfide, sodium dithionite, and ammonium sulfide; hydrazine; and formic acid. This reduction can also be caried out by contact reduction, in which a catalyst, such as platinum, Raney nickel, platinum black, and palladium-carbon, is used.

The amide-forming reaction (i.e., a reaction of an amino group and an acid chloride) is known as an extremely orthodox and high-yield reaction (see, for example, Shinjikkenkagakukoza 14 [II] p. 1142, Maruzen (1975)).

An amide compound can be easily obtained by mixing an amine compound with an acid chloride at a temperature of 0°C to 100°C in the presence of a tertiary amine, such as pyridine, using an amide-series solvent, such as N,N-dimethylfolmamide, as well as acetonitrile and ether.

The amide compound can also be obtained by the so-called Schotten-Baumann's reaction, in which an amine is added to an aqueous solution of an inorganic base (e.g., sodium hydroxide, sodium carbonate, potassium hydroxide, potassium carbonate, sodium acetate), and to the aqueous solution, is added an organic solvent (e.g., ethyl acetate or a halogen-series solvent, such as methylene chloride), if necessary, to solve the amine, and then an acid chloride is dropped into the resultant solution.

Specific examples of the compound of the present invention are illustrated below.

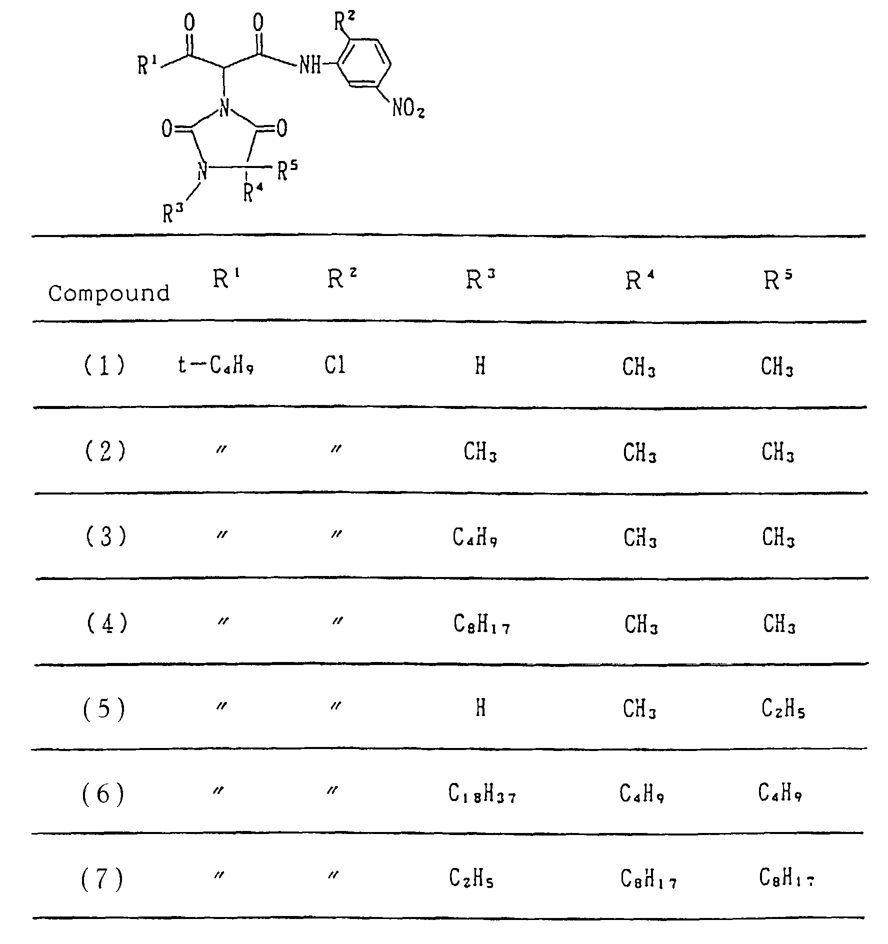

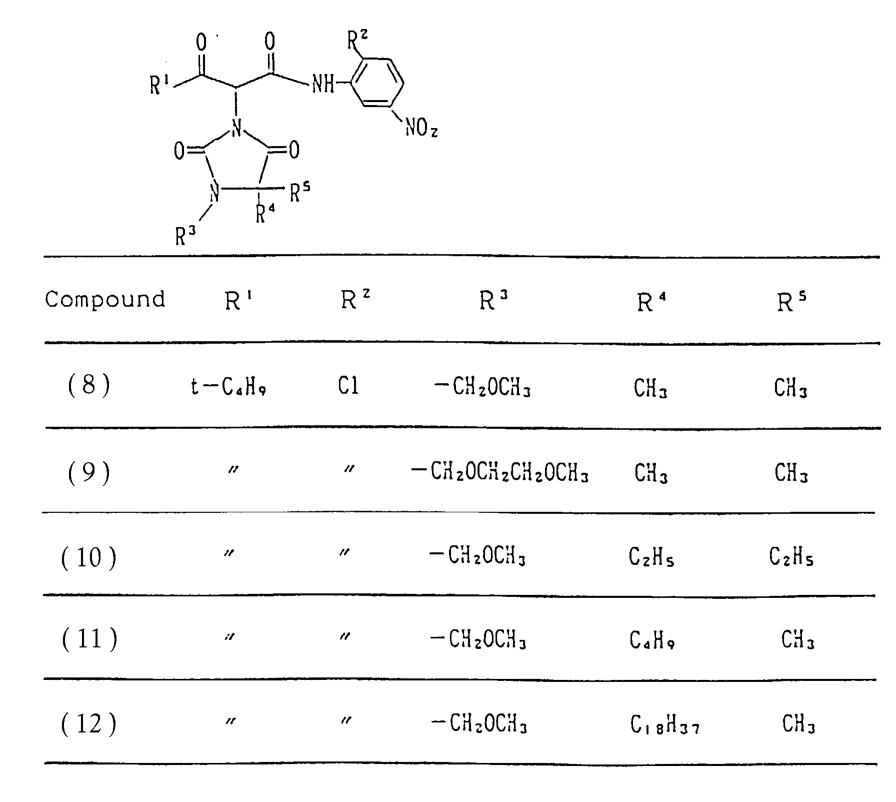

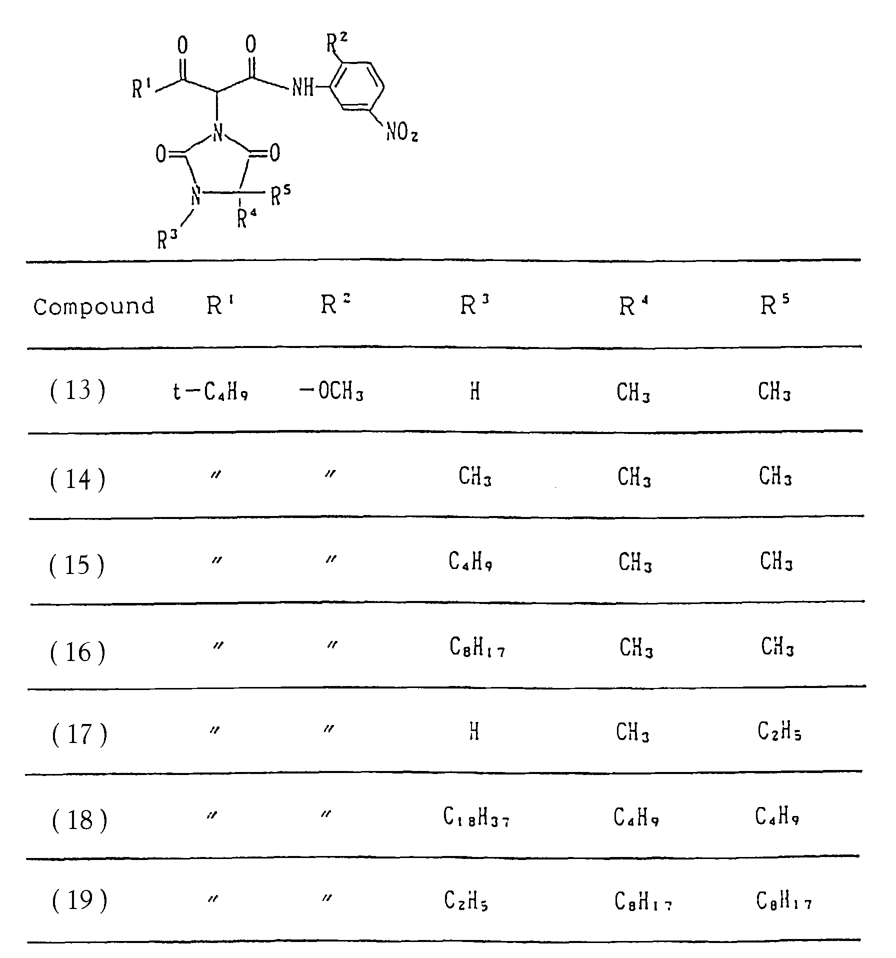

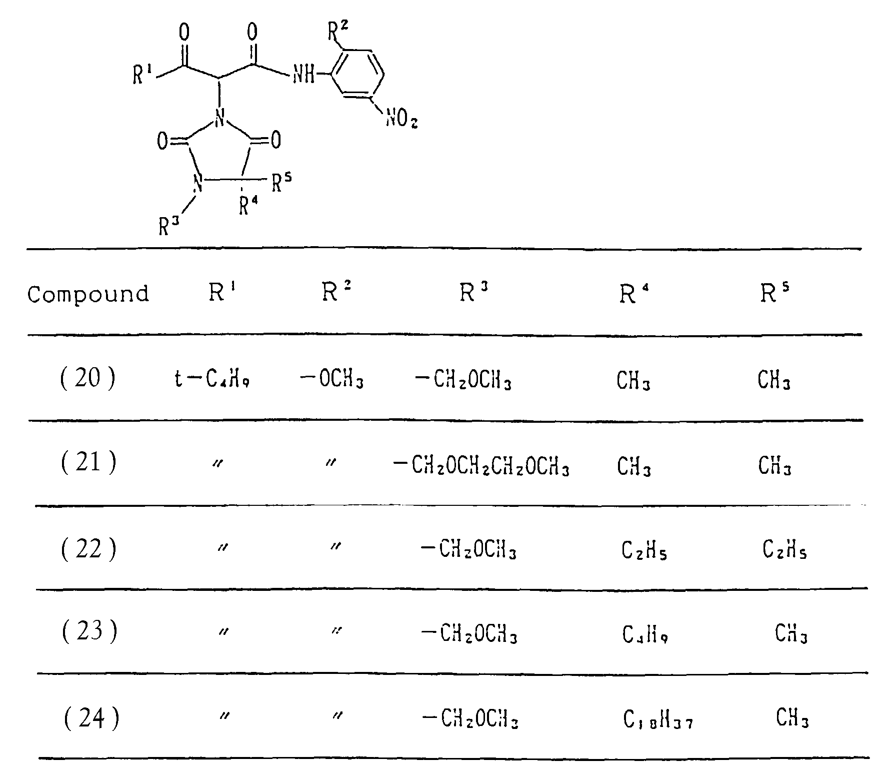

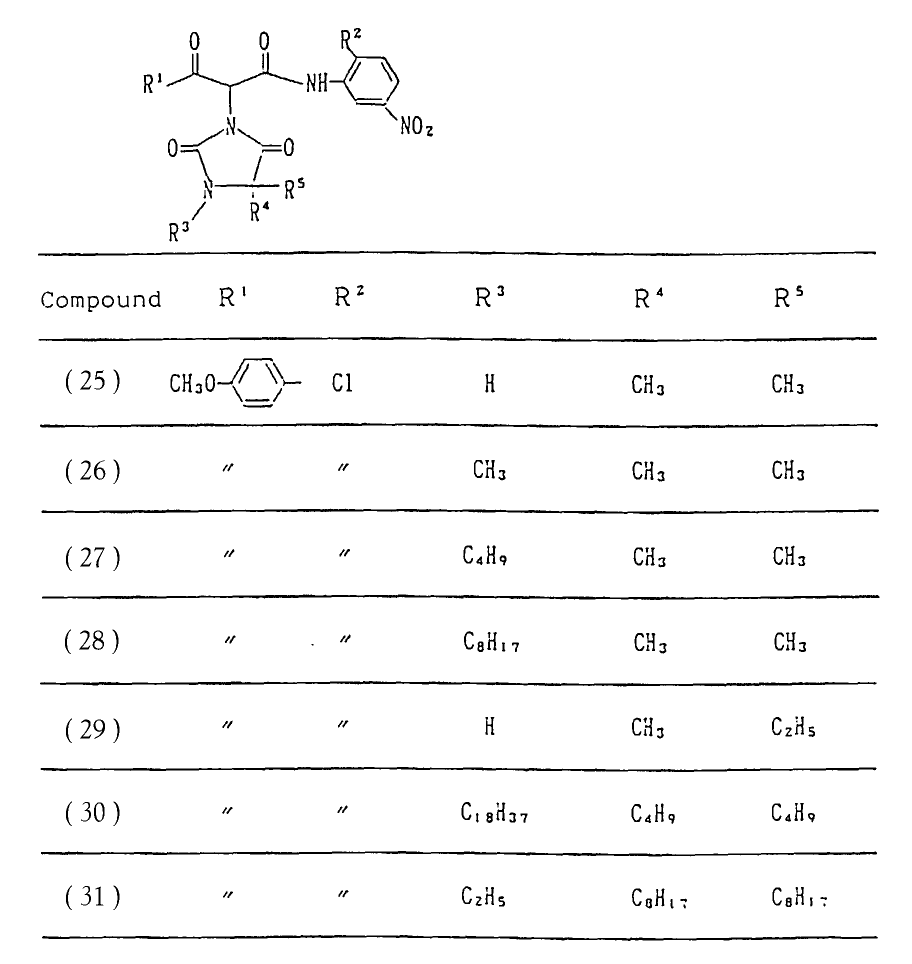

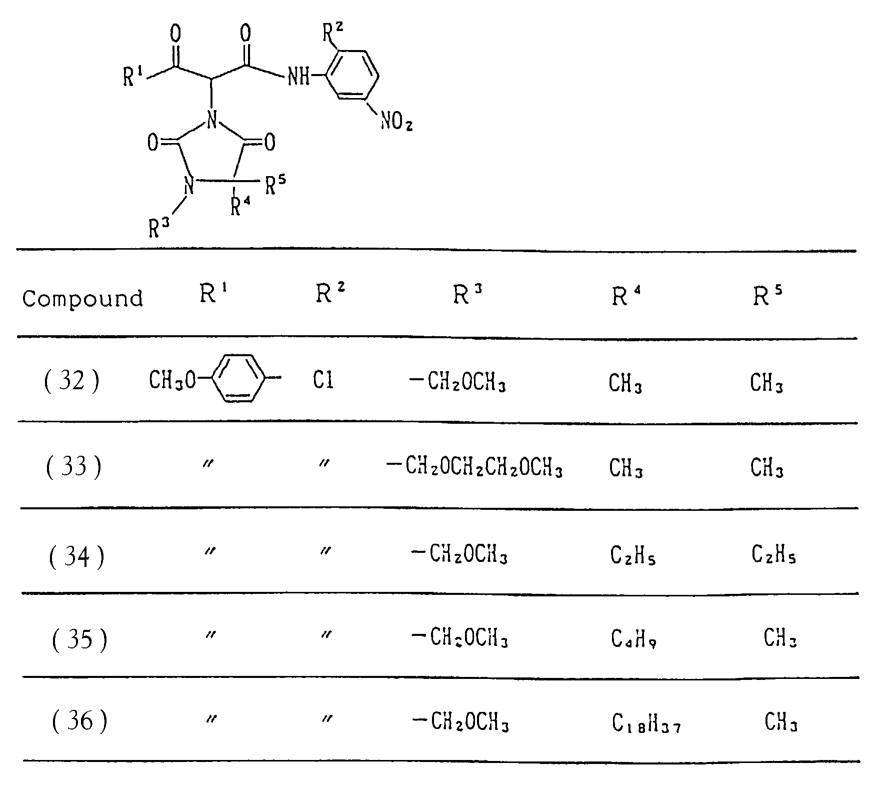

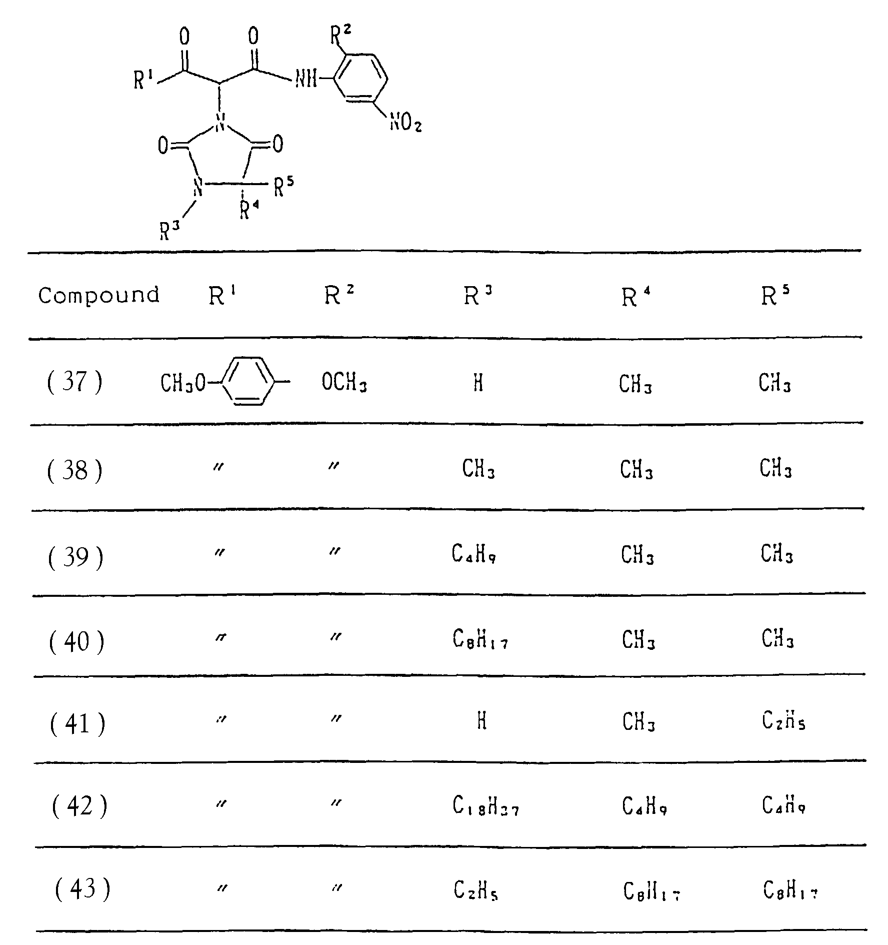

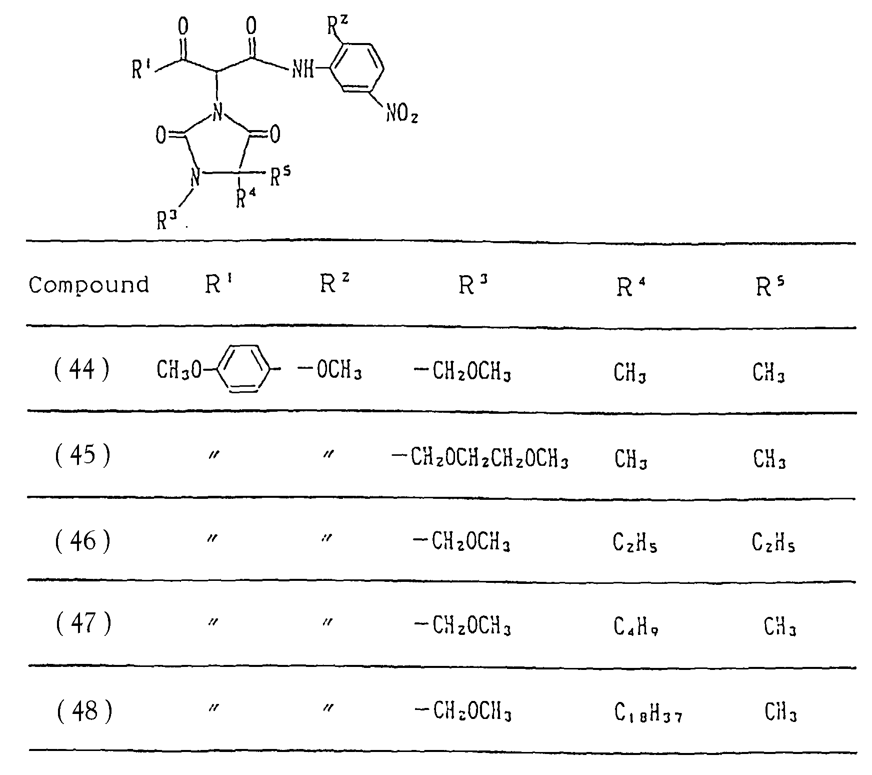

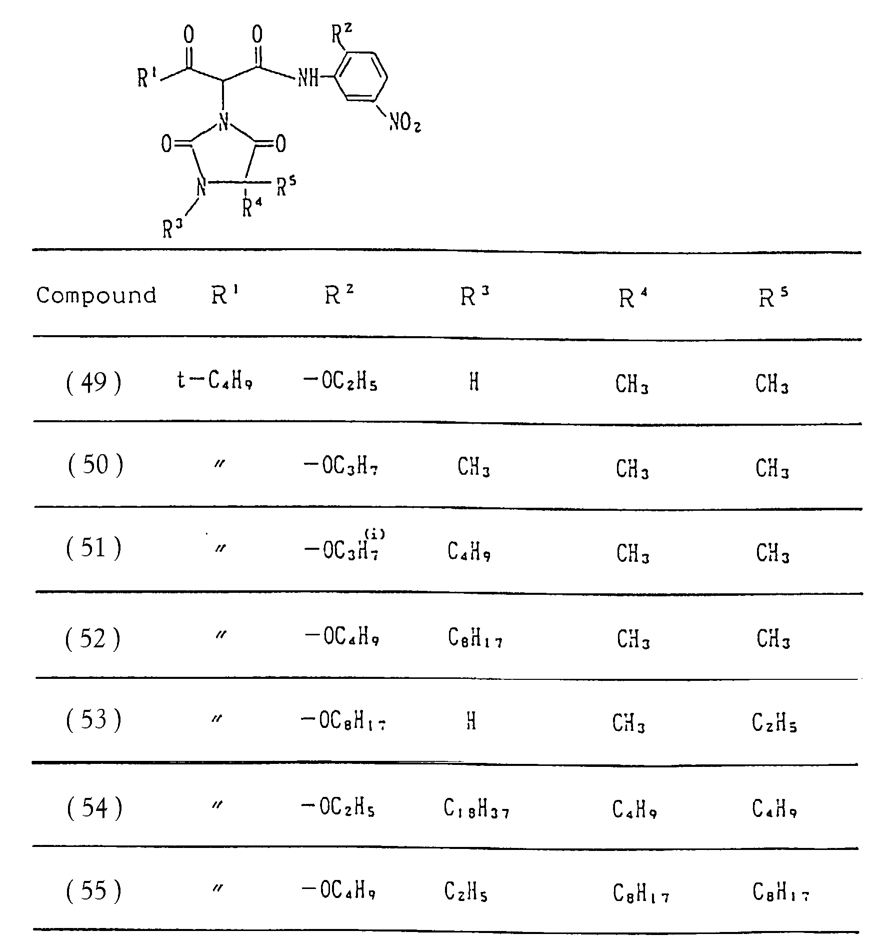

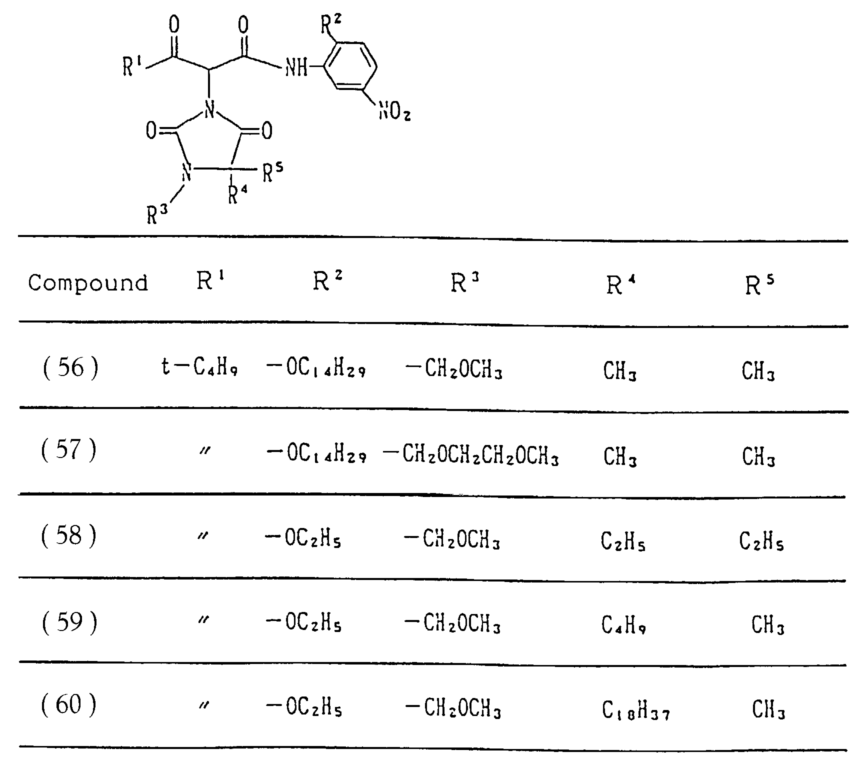

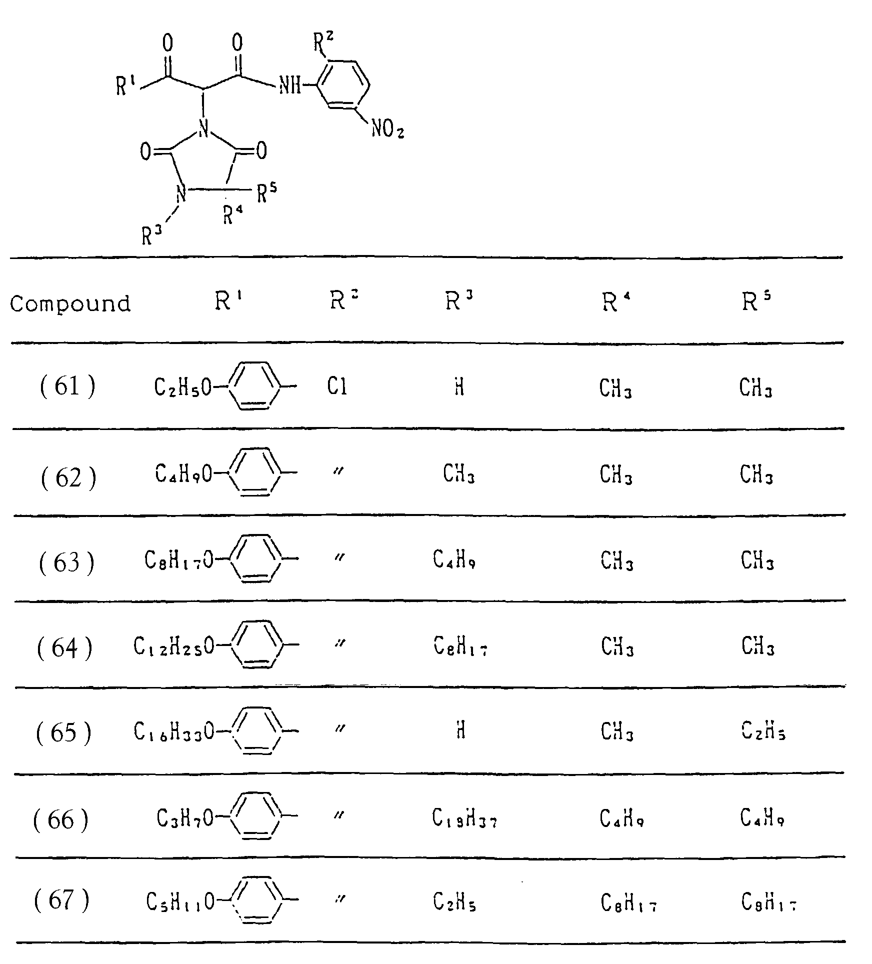

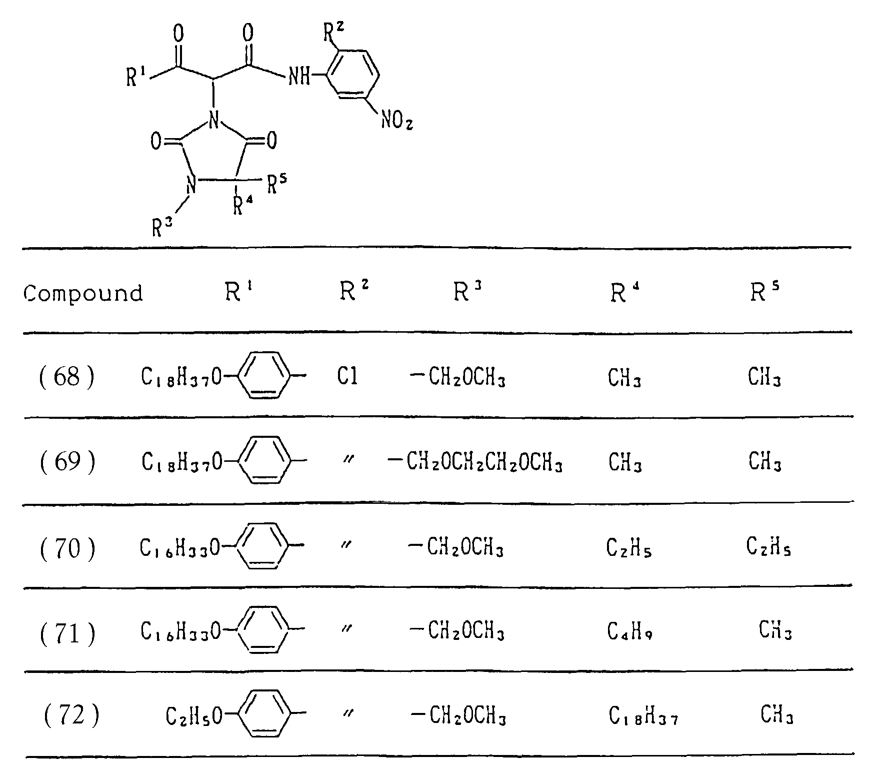

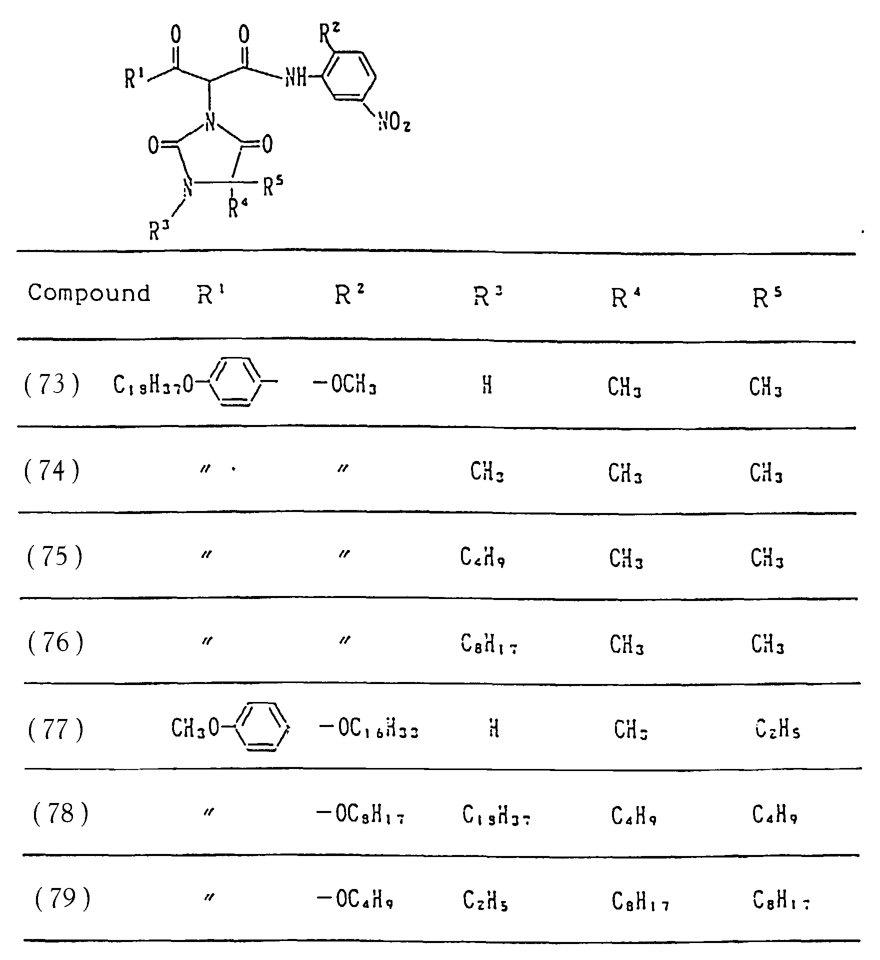

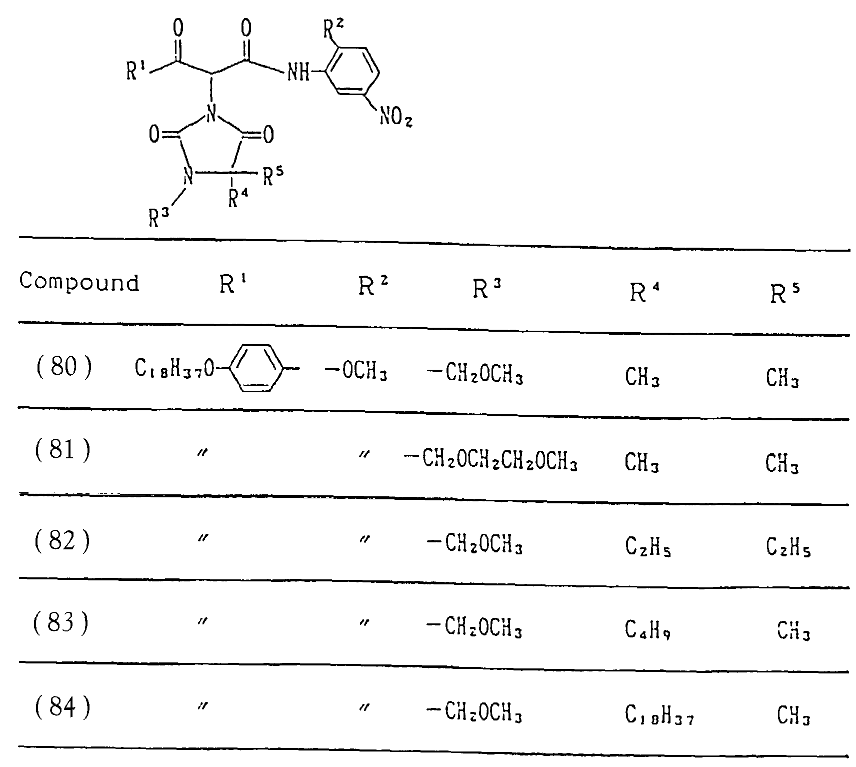

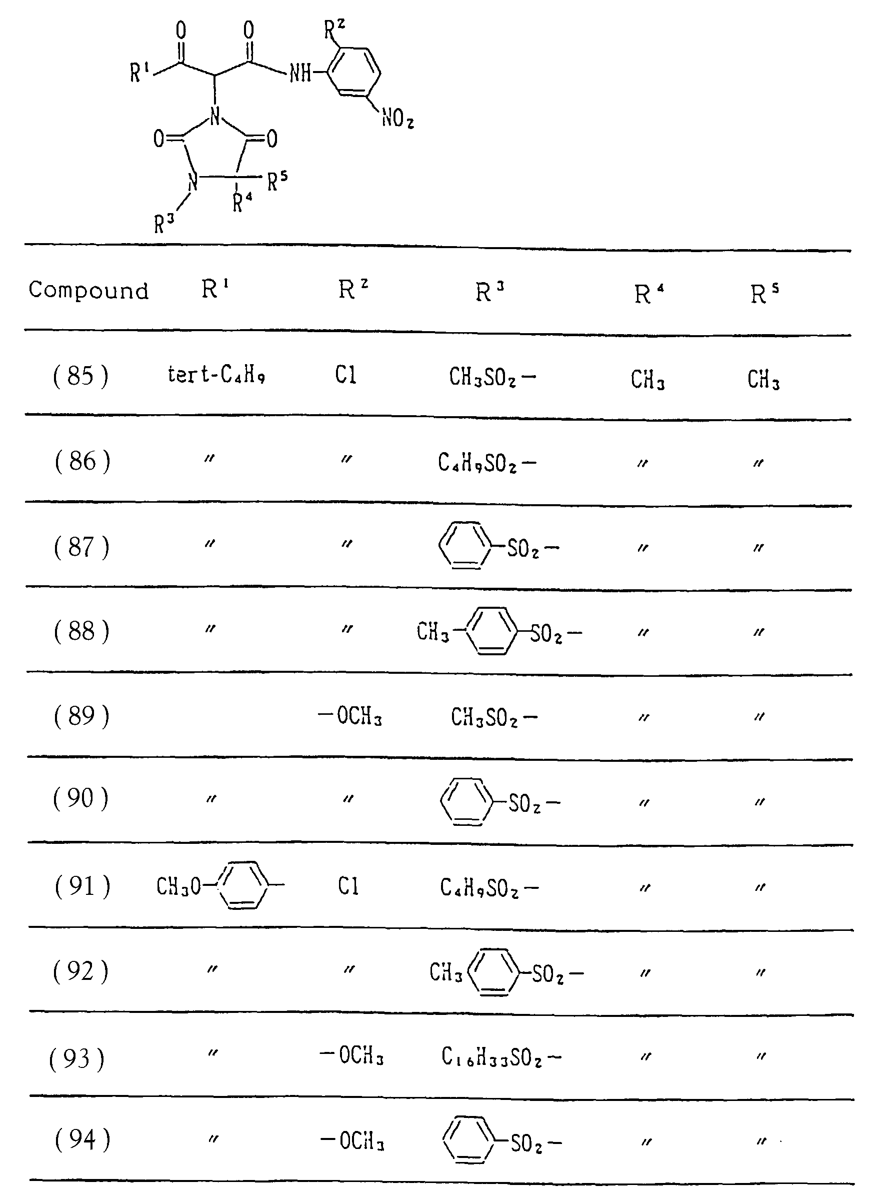

Photographic yellow couplers can be manufactured in short steps by using the compound of the present invention.

The method of manufacturing a photographic yellow coupler by using a synthetic intermediate of the present invention, has the following advantages:
(1) The occurrence and contamination of by-products that affect photographic properties can be avoided;
(2) Since a ballast group is introduced in the final step of making a yellow coupler, various kinds of yellow couplers useful in photography that differ from each other in terms of their ballast groups, can be readily synthesized from the intermediate of formula (I); and
(3) Since a low-molecular-weight coupling split-off group has been previously introduced in the intermediate of formula (I), the molar amounts of the intermediate to be used in the reaction for introducing a high-molecular-weight ballast group are relatively high, and this causes merits on cost.

Reduction reaction of a nitro group in the compound of formula (I) and subsequent acylation reaction can be carried out in continuous operation in a reactor, with a high yield, in addition to the merits described in (1). On the other hand, in a conventional method, in which the acylacetanilide compound 2 is acylated, halogenized, and then substituted, the inclusion of by-products, such as active halide-substituted compound (chloride- or bromide-substituted compound 3) and the compound 2 (four-equivalent couplers) having two hydrogen atoms at active sites, cannot be avoided. These by-products affect the photographic properties, such as amounts of formed dye and sensitivity, when they react with oxidized form of a color-developing agent. Further, a causative agent of fogging is sometimes generated by this conventional method.

In some yellow couplers that are widely used in photography, a so-called ballast group (oil-solubilizing group), which is a substituent derived from a long hydrocarbon chain carboxilic acid, is bonded in the coupler in a form of an acyl group. This ballast group usually has a high molecular weight. By introducing the ballast group at the final step of synthesis, the used amounts, i.e. molar numbers, of the intermediate for yellow coupler are made relatively large in one batch for manufacturing the yellow coupler. This means relatively large amounts of yellow coupler can be obtained in one batch according to the synthetic route of the present invention.

The present invention is described below in more detail based on the following examples.

### Preparation Example

First, tert-butyl (5,5-dimethylhydantoin-3-yl)methyl ketone (the substituted acetyl compound), as a primary raw material, was prepared as follows.

In 200 ml of N,N-dimethylfolmamide, were dispersed 38.4 g of 5,5-dimethylhydantoin (0.3 mol) and 46.0 g of potassium carbonate (0.33 mol). To the resultant mixture, was dropped 40.0 g (0.3 mol) of α-chloropinacolin (tert-butyl chloromethyl ketone), over 15 minutes at room temperature. After the dropping, the reaction mixture was heated on a steam bath for 2 hours, followed by cooling on an ice bath. To the reaction mixture, 700 ml of water was added drop-wise little by little. Precipitated crystals were filtered. As a result, 58.0 g of tert-butyl (5,5-dimethylhydantoin-3-yl)methyl ketone, as colorless crystals, was obtained. Yield: 85%.

Further, 4-chloro-5-phenoxycarbonylaminonitrobenzene, as another primary raw material, was prepared as follows.

In 80 ml of acetonitrile, 17.3 g (0.1 mol) of 2-chloro-5-nitroaniline was dispersed. To the resultant mixture, 13.2 ml (0.105 mol) of phenyl chlorocarbonate was added, followed by refluxing for 2.5 hours. To the reaction mixture, 50 mol of acetonitrile was added, followed by cooling to room temperature. Precipitated crystals were filtered. As a result, 23.1 g of 4-chloro-5-phenoxycarbonylaminonitrobenzene crystals was obtained. Yield: 79%.

To a dispersion of 0.22 g (5.5 milimoles) of sodium hydride (60%) in 10 ml of N,N-dimethylformamide, was added 1.13 g (5 milimoles) of tert-butyl (5,5-dimethylhydantoin-3-yl)methyl ketone as prepared above, in a nitrogen atomosphere, followed by stirring for 10 minutes at room temperature. To the resultant mixture, 0.58 ml (5 milimoles) of benzylchloride was added, followed by stirring for 2 hours at room temperature. After that, 0.44 g (11 milimoles) of sodium hydride was added to the reaction mixture, and then 5 ml of N,N-dimethylformamide solution containing 1.47 g (5 milimoles) of 4-chloro-5-phenoxycarbonylaminonitrobenzene as prepared above, was dropped into the mixture, on an ice bath, over 10 minutes. The reaction mixture was brought to room temperature, followed by stirring for 3 hours. After that, the reaction mixture was poured into an aqueous diluted hydrochloric acid solution, and the resultant solution was extracted with ethyl acetate. The extracted organic layer was washed twice with water, and then the solvent was extruded therefrom. After that, the resultant mixture was refined by silica gel column chromatography (n-hexane/ethyl acetate = 2/1), to obtain 1.71 g of a Compound of the formula (I) wherein R¹=t-C₄H₉, R²=Cl, R³=-CH₂-C₆H₅, R⁴=-CH₃ and R⁵=CH₃ (not falling under the scope of claim 1), in an amorphous form. Yield: 66%.

### EXAMPLE 1 Synthesis of Compound (1)

To a dispersion of 2.7 g (0.066 mol) of sodium hydride (60%) in 30 ml of N,N-dimethylfolmamide, was dropped 50 ml of N,N-dimethylfolmamide solution containing 6.8 g (0.03 mol) of tert-butyl (5,5-dimethylhydantoin-3-yl)methyl ketone and 8.8 g (0.03 mol) of 4-chloro-5-phenoxycarbonylaminonitrobenzene, prepared in the same manner as in Preparation Example, over 2 hours on an ice bath in a nitrogen atmosphere. Then the reaction mixture as such was brought to room temperature and was stood for 2 nights. After that, the reaction mixture was poured into ice water and neutralized with diluted hydrochloric acid. Then, the resultant solution was extracted with ethyl acetate, the resultant organic layer was washed with water, dried, concentrated, and then refined by a silica gel column chromatography (n-hexane/ethyl acetate = 2/1), to obtain 0.69 g of compound (1) in an amorphous form. Yield: 5.4%. Thus obtained compound (1) was high in purity containing little by-product.
¹H-NMR (300 MHz : CDCl₃)
- δ ppm: 1.30 (s, 9H)
1.54 (s, 3H)
1.58 (s, 3H)
5.69 (s, 1H)
6.25 (s, 1H)
7.57 (d, 1H, J=9.3 Hz)
7.96 (dd, 1H, J=9.3 Hz, 3.3Hz)
9.25 (d, 1H, J=3.3 Hz)
10.0 (s, 1H)

### EXAMPLE 2 Synthesis of Compound (8)

Compound (8) was prepared in the same manner as Example 1, except that chloromethyl methyl ether was used in place of benzylchloride. Yield: 75%.
¹H-NMR (300 MHz : CDCl₃)
- δ ppm: 1.29 (s, 9H)
1.56 (s, 9H)
1.61 (s, 3H)
3.40 (s, 3H)
4.90 (s, 2H)
5.72 (s, 1H)
7.57 (d, 1H, J=8.7 Hz)
7.96 (dd, 1H, J=8.7 Hz, 2.7 Hz)
9.26 (d, 1H, J=2.7 Hz)
10.05 (s, 1H)

### EXAMPLE 3 Synthesis of Compound (12)

Compound (12) was prepared in the same manner as the Preparation Example, except that 2-methoxy-5-nitroaniline was used in place of 2-chloro-5-nitroaniline.
Amorphous.
¹H-NMR (300 MHz : CDCl₃)
- δ ppm: 1.32 (s, 9H)
1.53 (s, 3H)
1.58 (s, 3H)
4.03 (s, 3H)
5.70 (s, 1H)
6.15 (s, 1H)
6.95 (d, 1H, J=10.0 Hz)
8.02 (dd, 1H, J=10.0 Hz, 2.7 Hz)
9.17 (d, 1H, J=2.7 Hz)
9.40 (s, 1H)

### EXAMPLE 4 Synthesis of Compound (19)

Compound (19) was prepared in almost the same manner as the above Examples. Yield: 72%.
Amorphous.
¹H-NMR (300 MHz : CDCl₃)
- δ ppm: 1.28 (s, 9H)
1.56 (s, 3H)
1.60 (s, 3H)
3.40 (s, 3H)
4.00 (s, 3H)
4.90 (s, 2H)
5.70 (s, 1H)
6.93 (d, 1H, J=8.0 Hz)
8.02 (dd, 1H, J=8.0 Hz, 2.7 Hz)
9.22 (d, 1H, J=2.7 Hz)
9.70 (s, 1H)

### EXAMPLE 5 Synthesis of Compound (84)

Compound (84) was prepared in the same manner as Example 1, except that methanesulfonylchloride was used in place of benzylchloride. Yield: 68%.
Amorphous.
¹H-NMR (300 MHz : CDCl₃)
- δ ppm: 1.30 (s, 9H)
1.83 (s. 3H)
1.88 (s, 3H)
3.43 (s, 3H)
5.67 (s, 1H)
7.58 (d, 1H, J=9.3 Hz)
8.00 (dd, 1H, J=9.3 Hz, 2.7 Hz)
9.20 (d, 1H, J=2.7 Hz)
9.73 (s, 1H)

### EXAMPLE 6 Synthesis of Compound (87)

Compound (87) was prepared in the same manner as Example 1, except that p-toluenesulfonylchloride was used in place of benzylchloride. Yield: 70%.
Amorphous.
¹H-NMR (300 MHz : CDCl₃)
- δ ppm: 1.22 (s, 9H)
1.83 (s, 3H)
1.90 (s, 3H)
2.44 (s, 3H)
5.55 (s, 1H)
7.3 (m, 2H)
7.52 (d, 1H, J=9.0 Hz)
7.95 (m, 3H)
9.13 (d, 1H, J=2.7 Hz)
9.65 (s, 1H)

### EXAMPLE 7 Synthesis of Compound (1)

(A different condition from that of Example 1; A method of using Compound (87))

In 100 ml of methanol, was dissolved 5.80 g (0.01 mol) of Compound (87) as prepared in Example 6, and then 10.0 ml (0.05 mol) of methanol solution (28%) containing sodium methoxide was added thereto, followed by refluxing for 1 hour.

The reaction mixture was brought to room temperature. After that, the reaction mixture was acidified with hydrochloric acid, extracted with ethyl acetate, the resultant organic layer was washed with water, dried, concentrated, and then refined by a silica gel column chromatography (n-hexane/ethyl acetate = 2/1), to obtain 3.14 g of Compound (1) in an amorphous form. Yield: 74%.

## Claims

1. A hydantoin-substituted acylacetanilide compound represented by general formula (I): 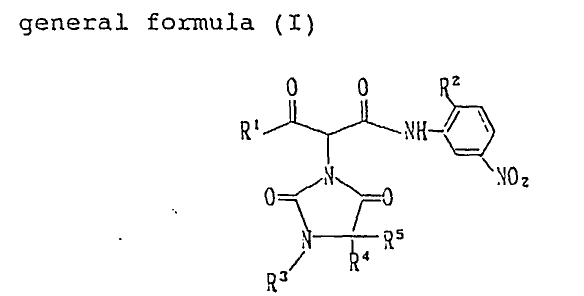 wherein R¹ represents a tert-butyl group or a p-alkoxyphenyl group, R² represents a chlorine atom or an alkoxy group, R³ represents a hvdroqen atom, an unsubstituted or alkoxy-substituted alkyl group, an alkylsulfonyl group, or an arylsulfonyl group, R⁴ represents an alkyl group, and R⁵ represents an alkyl group.

2. The hydantoin-substituted acylacetanilide compound as defined in claim 1, in which the compound of the formula (I) is represented by the formula (1), (5), (13), (17), (25), (29), (37), (49), (53), (61), (65), (73) or (77):

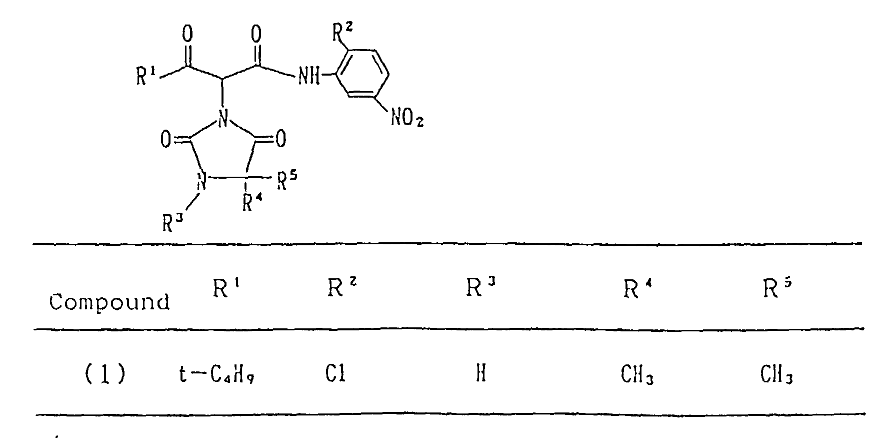

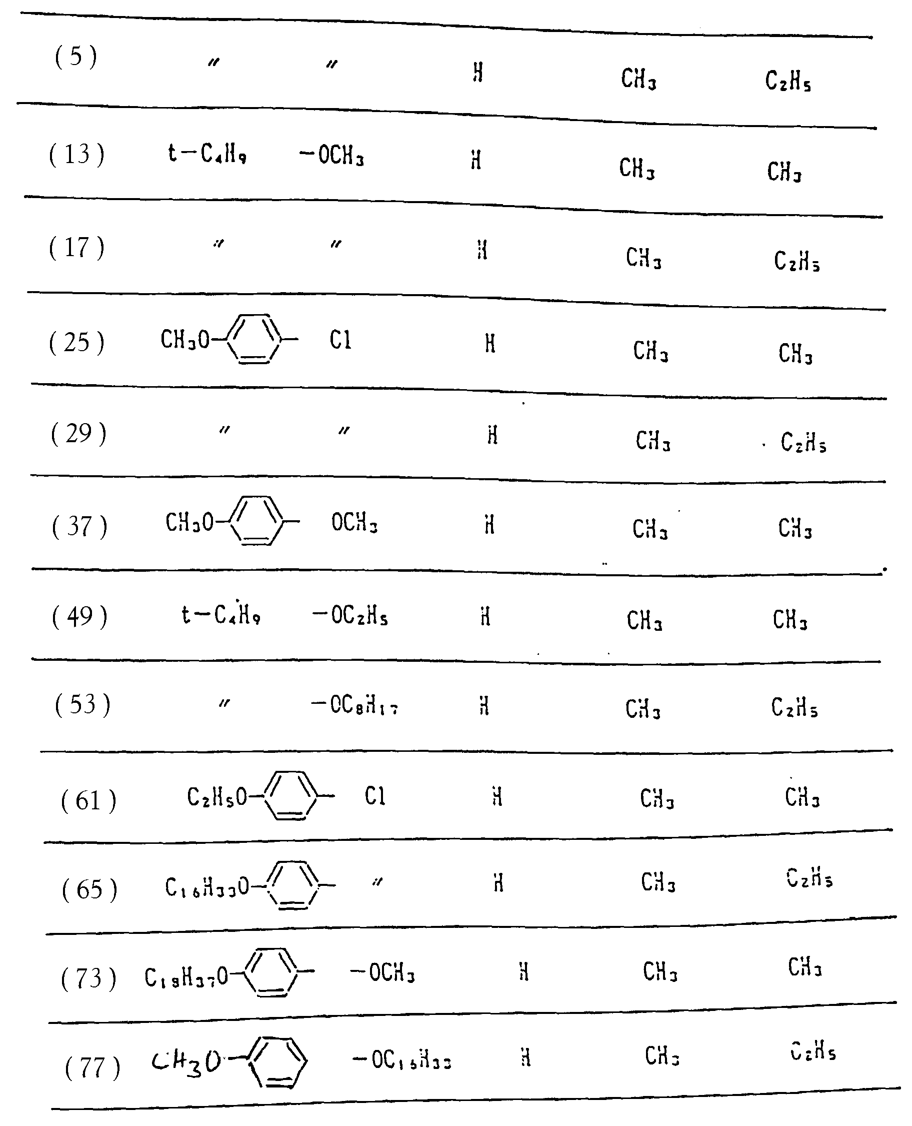

3. The hydantoin-substituted acylacetanilide compound as claimed in claim 1, wherein R¹ represents a t-butyl group, R² represents a chlorine atom or a methoxy group, R⁴ represents a methyl group, R⁵ represents a methyl group, and R³ represents a hydrogen atom.

4. A method of manufacturing a hydantoin-substituted acylacetanilide compound represented by general formula (I): 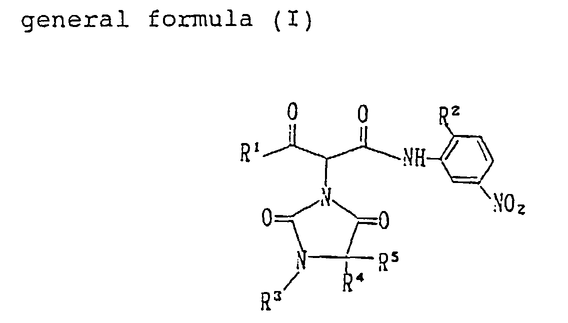 wherein R¹ represents a tert-butyl group or a p-alkoxyphenyl group, R² represents a chlorine atom or an alkoxy group, R³ represents a hydrogen atom, an unsubstituted or alkoxy-substituted alkyl group, an alkylsulfonyl group, or an arylsulfonyl group, R⁴ represents an alkyl group, and R⁵ represents an alkyl group, comprising reacting a hydantoin-substituted acetyl compound represented by general formula (III): 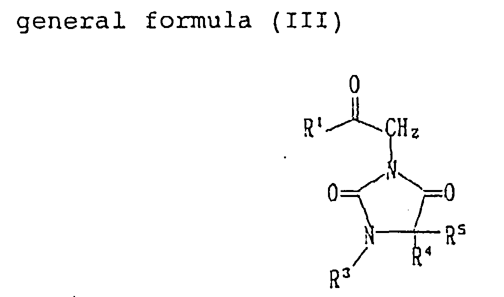 wherein R¹, R³, R⁴, and R⁵ each have the same meanings as those of general formula (I), with a urethane compound represented by general formula (IV): 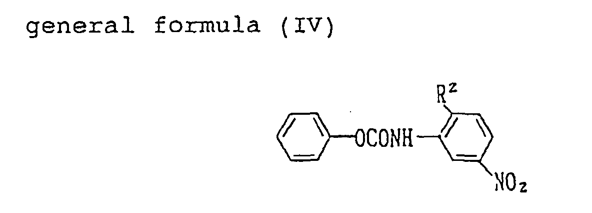 wherein R² has the same meaning as that of general formula (I), in the presence of a base.

5. The method of manufacturing a hydantoin-substituted acylacetanilide compound as claimed in claim wherein the base is a sodium hydride.

6. A method of manufacturing a compound represented by general formula (II): 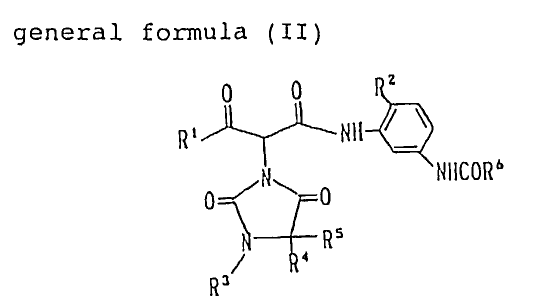 wherein R¹ represents a tert-butyl group or a p-alkoxyphenyl group, R² represents a chlorine atom or an alkoxy group, R³ represents a hydrogen atom, an unsubstituted or alkoxy-substituted alkyl group, an alkylsulfonyl group, or an arylsulfonyl group, R⁴ represents an alkyl group, R⁵ represents an alkyl group, and R⁶ represents a substituted or unsubstituted alkyl group, comprising reacting a hydantoin-substituted acetyl compound represented by general formula (III): 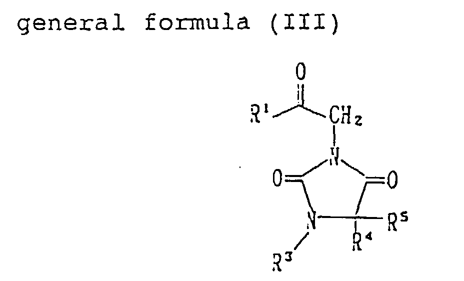 wherein R¹, R³, R⁴, and R⁵ each have the same meanings as those of general formula (II), with a urethane compound represented by general formula (IV): 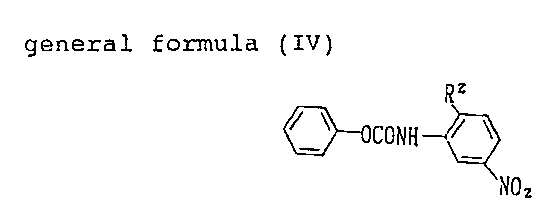 wherein R² has the same meaning as that of general formula (II), in the presence of a base, to obtain a hydantoin-substituted acylacetanilide compound represented by general formula (I): 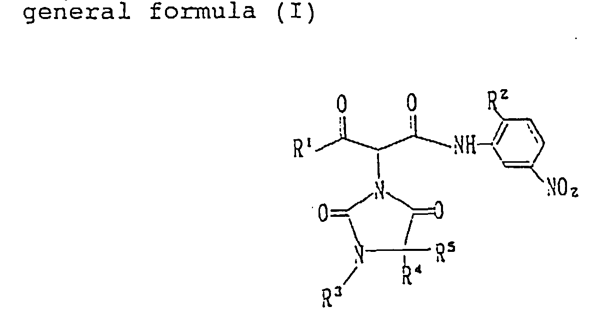 wherein R¹, R², R³, R⁴, and R⁵ each have the same meanings as those of general formula (II), then reducing a nitro group of the hydantoin-substituted acylacetanilide compound represented by general formula (I) to an amino group, and then reacting the resulting amino compound with an acid halide represented by general formula (V):
general formula (V) R⁶COX
wherein R⁶ has the same meaning as that of general formula (II), and X represents a halogen atom.

## Patentansprüche

1. Hydantoin-substituierte Acylacetanilid-Verbindung mit der allgemeinen Formel (I): 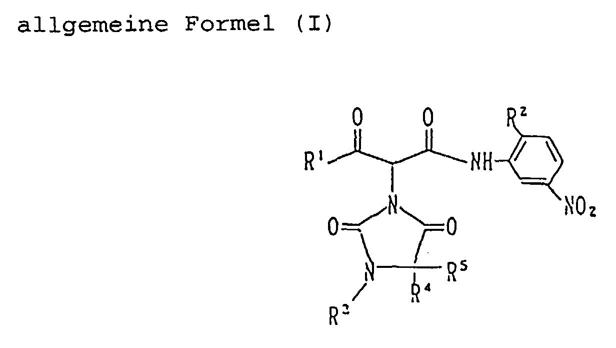 worin R¹ eine tert-Butyl-Gruppe oder p-Alkoxyphenyl-Gruppe ist, R² ein Chloratom oder eine Alkoxy-Gruppe ist, R³ ein Wasserstoffatom, eine unsubstituierte oder Alkoxy-substituierte Alkyl-Gruppe, Alkylsulfonyl-Gruppe oder Arylsulfonyl-Gruppe ist, R⁴ eine Alkyl-Gruppe ist und R⁵ eine Alkyl-Gruppe ist.

2. Hydantoin-substituierte Acylacetanilid-Verbindung nach Anspruch 1, worin die Verbindung der Formel (I) durch die Formel (1), (5), (13), (17), (25), (29), (37), (49), (53), (61), (65), (73) oder (77) dargestellt ist:

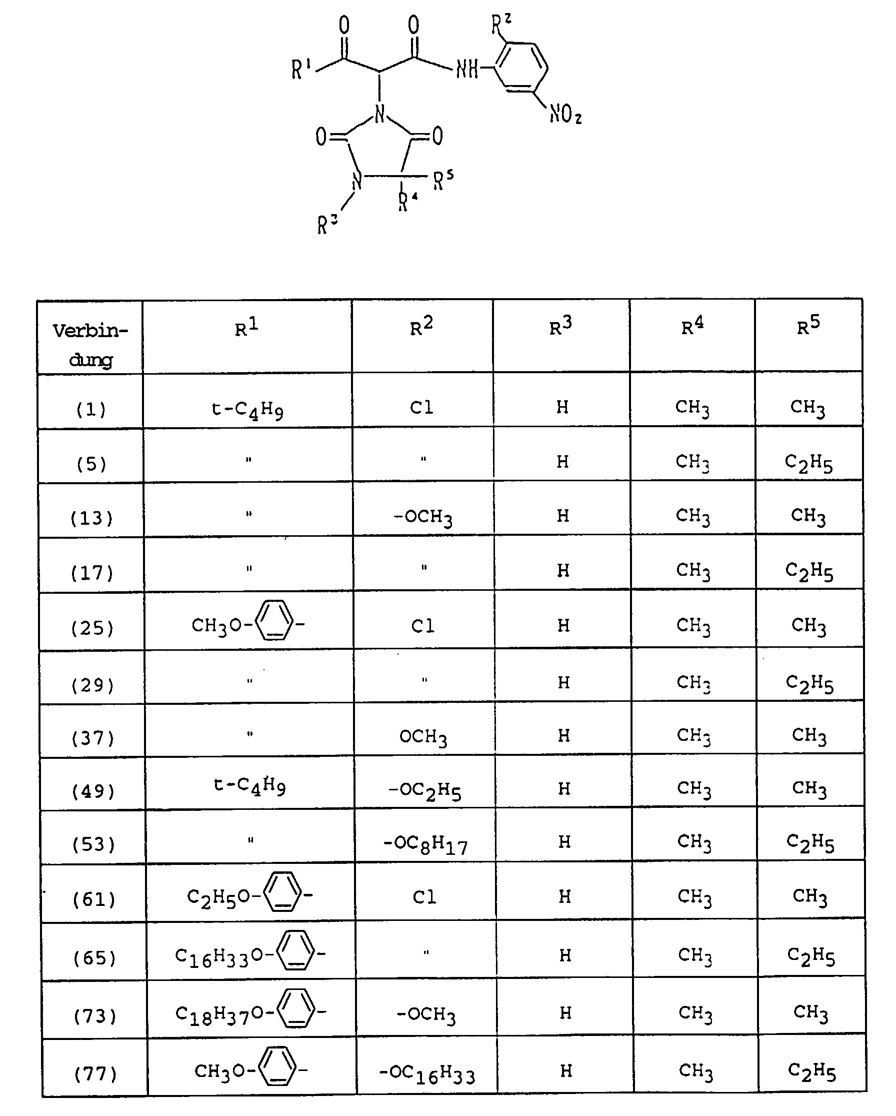

3. Hydantoin-substituierte Acylacetanilid-Verbindung nach Anspruch 1, worin R¹ eine t-Butyl-Gruppe, R² ein Chloratom oder eine Methoxy-Gruppe, R⁴ eine Methyl-Gruppe, R⁵ eine Methyl-Gruppe und R³ ein Wasserstoffatom sind.

4. Verfahren zur Herstellung einer Hydantoin-substituierten Acylacetanilid-Verbindung mit der allgemeinen Formel (I): 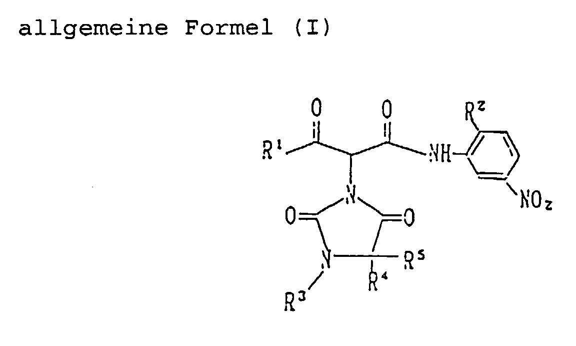 worin R¹ eine tert-Butyl-Gruppe oder p-Alkoxyphenyl-Gruppe ist, R² ein Chloratom oder eine Alkoxy-Gruppe ist, R³ ein Wasserstoffatom, eine unsubstituierte oder Alkoxy-substituierte Alkyl-Gruppe, Alkylsulfonyl-Gruppe oder Arylsulfonyl-Gruppe ist, R⁴ eine Alkyl-Gruppe ist und R⁵ eine Alkyl-Gruppe ist, umfassend die Reaktion einer Hydantoin-substituierten Acetyl-Verbindung mit der allgemeinen Formel (III): 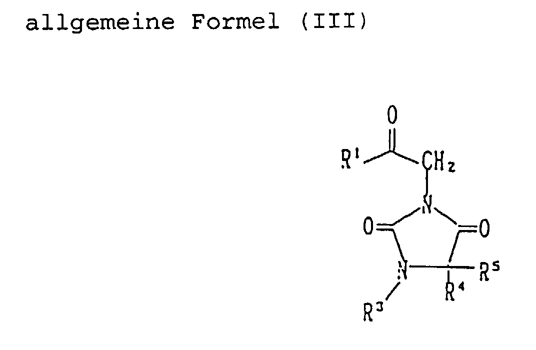 worin R¹, R³, R⁴ und R⁵ jeweils die gleichen Bedeutungen wie bei der allgemeinen Formel (I) aufweisen, mit einer Urethan-Verbindung mit der allgemeinen Formel (IV): 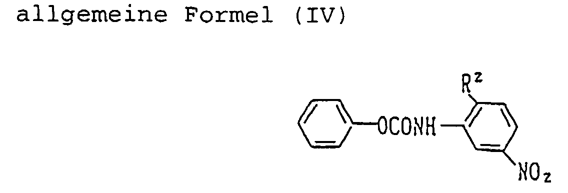 worin R² die gleiche Bedeutung wie bei der allgemeinen Formel (I) aufweist, in der Gegenwart einer Base.

5. Verfahren zur Herstellung einer Hydantoin-substituierten Acylacetanilid-Verbindung nach Anspruch 4, worin die Base Natriumhydrid ist.

6. Verfahren zur Herstellung einer Verbindung, dargestellt durch die allgemeine Formel (II): 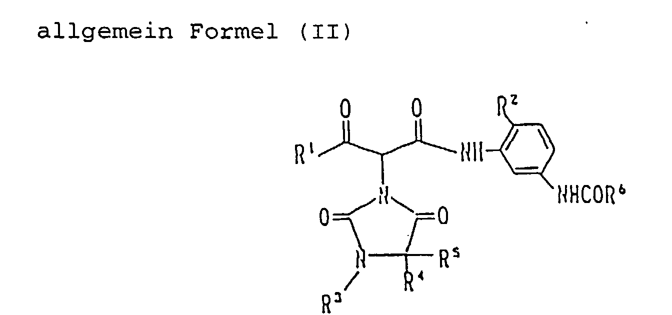 worin R¹ ein tert-Butyl-Gruppe oder ein p-Alkoxyphenyl-Gruppe, R² ein Chloratom oder ein Alkoxy-Gruppe, R³ ein Wasserstoffatom, eine unsubstituierte oder Alkoxy-substituierte Alkyl-Gruppe, Alkylsulfonyl-Gruppe oder Arylsulfonyl-Gruppe, R⁴ eine Alkyl-Gruppe, R⁵ eine Alkyl-Gruppe und R⁶ eine substituierte oder unsubstituierte Alkyl-Gruppe sind, umfassend die Reaktion einer Hydantoin-substituierten Acetyl-Verbindung mit der allgemeinen Formel (III): 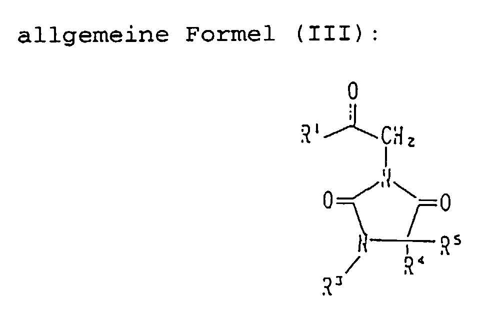 worin R¹, R³, R⁴ und R⁵ jeweils die gleichen Bedeutungen wie bei der allgemeinen Formel (II) haben, mit einer Urethan-Verbindung, dargestellt durch die allgemeine Formel (IV): 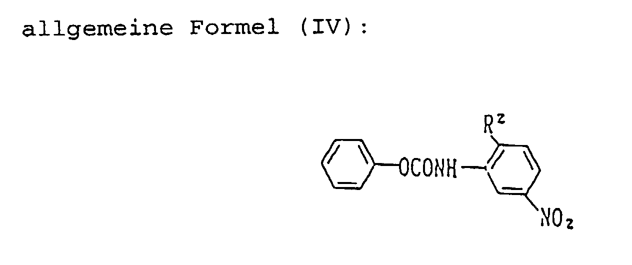 worin R² die gleiche Bedeutung wie bei der allgemeinen Formel (II) hat, in der Gegenwart einer Base, unter Erhalt einer Hydantoin-substituierten Acylacetanilid-Verbindung mit der allgemeinen Formel (I): 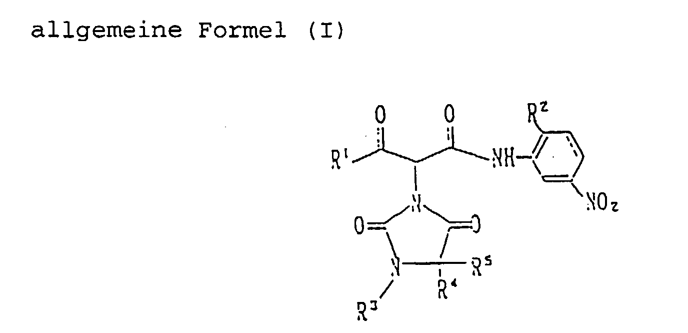 worin R¹, R², R³, R⁴ und R⁵ jeweils die gleichen Bedeutungen wie bei der allgemeinen Formel (II) aufweisen, anschließende Reduktion der Nitro-Gruppe der Hydantoin-substituierten Acylacetanilid-Verbindung mit der allgemeinen Formel (I) in eine Amino-Gruppe und anschließende Reaktion der resultierenden Aminoverbindung mit einem Säurehalogenid mit der allgemeinen Formel (V):
allgemeine Formel (V) R⁶COX
worin R⁶ die gleiche Bedeutung wie bei der allgemeinen Formel (II) hat und X ein Halogenatom bedeutet.

## Revendications

1. Composé acylacétanilide hydantoïne-substitué représenté par la formule générale (I) : 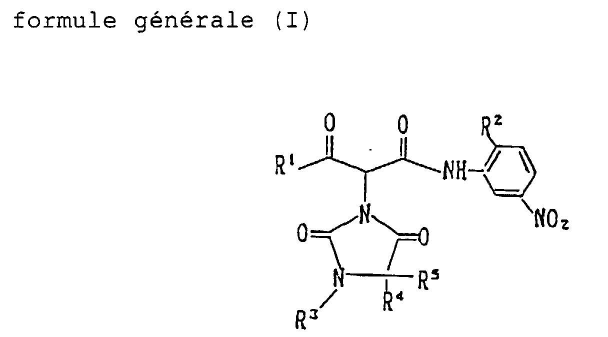 dans laquelle R¹ représente un groupe tert-butyle ou un groupe p-alcoxy-phényle, R² représente un atome de chlore ou un groupe alcoxy, R³ représente un atome d'hydrogène, un groupe alkyle non substitué ou alcoxy-substitué, un groupe alkyl-sulfonyle ou un groupe aryl-sulfonyle, R⁴ représente un groupe alkyle et R⁵ représente un groupe alkyle.

2. Composé acylacétanilide hydantoïne-substitué comme défini dans la revendication 1, où le composé de la formule (I) est représenté par la formule (1), (5), (13), (17), (25), (29), (37), (49), (53), (61), (65), (73) ou (77) :

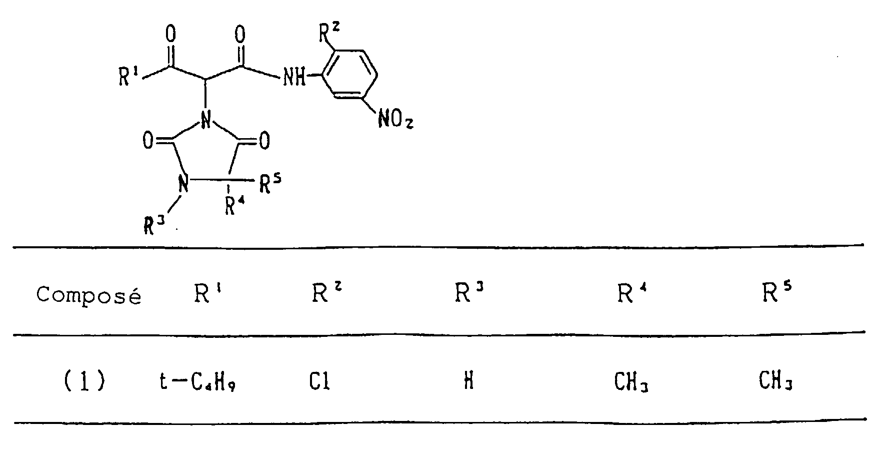

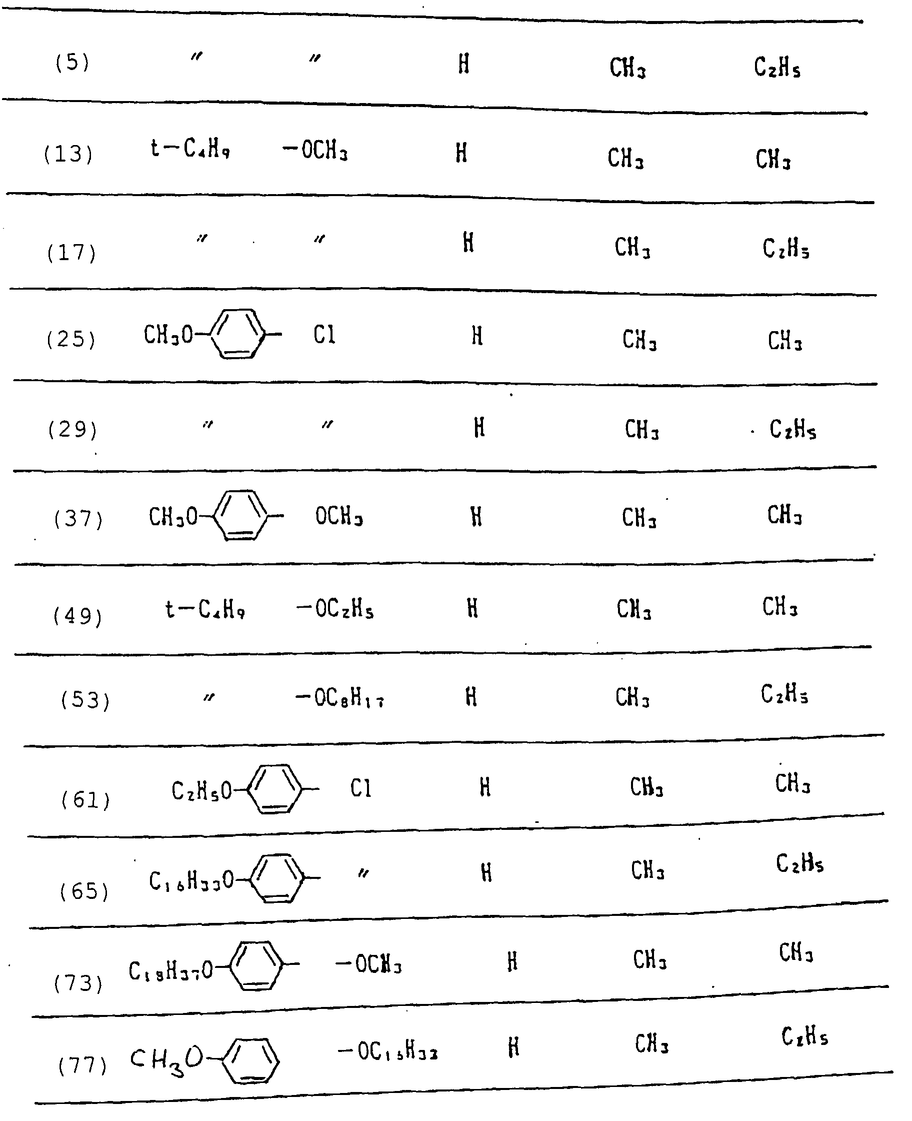

3. Composé acylacétanilide hydantoïne-substitué selon la revendication 1, où R¹ représente un groupe t-butyle, R² représente un atome de chlore ou un groupe méthoxy, R⁴ représente un groupe méthyle, R⁵ représente un groupe méthyle et R³ représente un atome d'hydrogène.

4. Procédé de préparation d'un composé acylacétanilide hydantoïne-substitué représenté par la formule générale (I) : 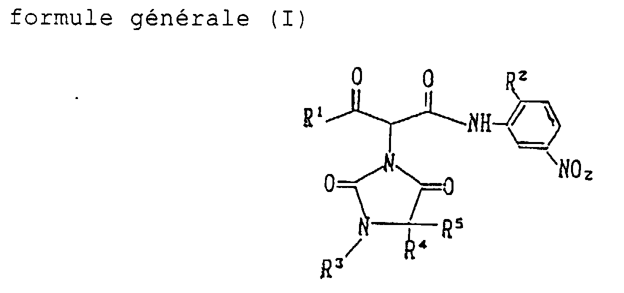 dans laquelle R¹ représente un groupe tert-butyle ou un groupe p-alcoxy- phényle, R² représente un atome de chlore ou un groupe alcoxy, R³ représente un atome d'hydrogène, un groupe alkyle non substitué ou alcoxy-substitué, un groupe alkyl-sulfonyle ou un groupe aryl-sulfonyle, R⁴ représente un groupe alkyle et R⁵ représente un groupe alkyle comprenant de faire réagir un composé acétyle hydantoïne-substitué représenté par la formule générale (III) : 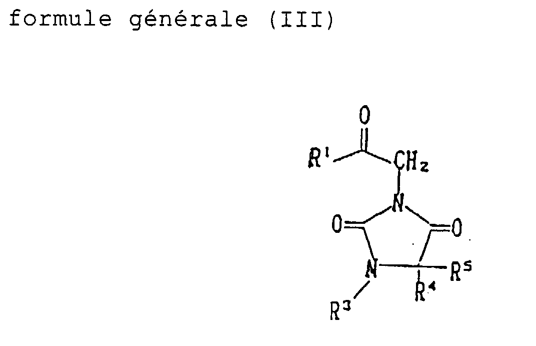 dans laquelle R¹, R³, R⁴ et R⁵ ont chacun la même signification que celle indiquée pour la formule générale (I), avec un composé uréthane représenté par la formule générale (IV) : 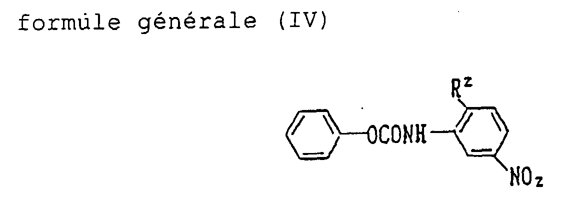 dans laquelle R² a la même signification que celle indiquée pour la formule générale (I), en présence d'une base.

5. Procédé de préparation d'un composé acylacétanilide hydantoïne-substitué selon la revendication 4, dans lequel la base est l'hydrure de sodium.

6. Procédé de préparation d'un composé représenté par la formule générale (II) : 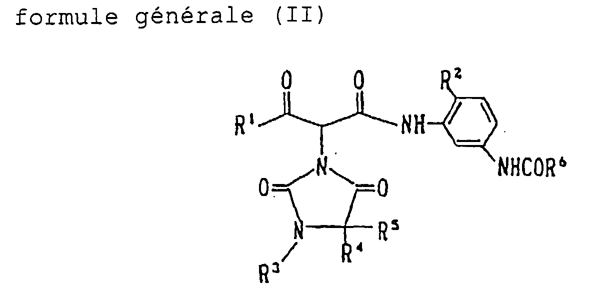 dans laquelle R¹ représente un groupe tert-butyle ou un groupe p-alcoxy-phényle, R² représente un atome de chlore ou un groupe alcoxy, R³ représente un atome d'hydrogène, un groupe alkyle non substitué ou alcoxy-substitué, un groupe alkyl-sulfonyle ou un groupe aryl-sulfonyle, R⁴ représente un groupe alkyle, R⁵ représente un groupe alkyle et R⁶ représente un groupe alkyle substitué ou non substitué, comprenant les étapes comprenant de faire réagir un composé acétyle hydantoïne-substitué représenté par la formule générale (III) : 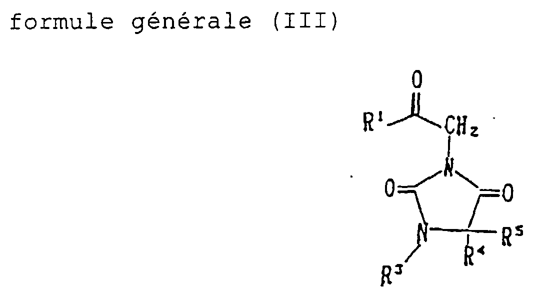 dans laquelle R¹, R³, R⁴ et R⁵ ont chacun la même signification que celle indiquée pour la formule générale (II), avec un composé uréthane représenté par la formule générale (IV) : 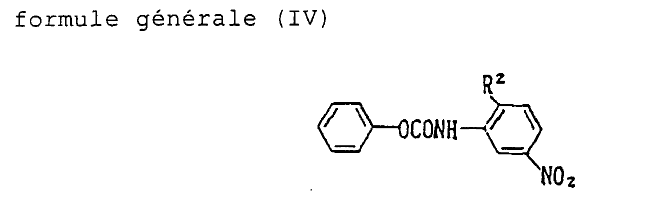 dans laquelle R² a la même signification que celle indiquée pour la formule générale (II), en présence d'une base, pour obtenir un composé acylacétanilide hydantoïne-substitué représenté par la formule générale (I) : 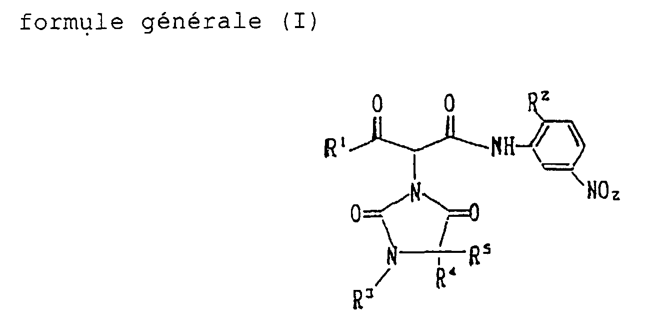 dans laquelle R¹, R², R³, R⁴ et R⁵ ont chacun la même signification que celle indiquée pour la formule générale (II) et ensuite de réduire un groupe nitro du composé acylacétanilide hydantoïne-substitué représenté par la formule générale (I) en un groupe amino, puis de faire réagir le composé amino résultant avec un halogénure d'acide, représenté par la formule générale (V) :
formule générale (V) R⁶COX
dans laquelle R⁶ a la même signification que celle indiquée pour la formule générale (II) et X représente un atome d'halogène.
